# EUROPÄISCHE PATENTANMELDUNG

(11) **EP 4 821 444 A1**
(43) Veröffentlichungstag der Anmeldung: **14.10.2026**
(21) Anmeldenummer: 26168461.7
(22) Anmeldetag: 27.03.2026
(51) Int. Cl.: A61B 8/08, A61B 8/12, A61B 8/00

(54) **VERFAHREN UND SYSTEM ZUM ERMITTELN EINER POSITION EINER KANÜLENSPITZE IN EINEM ULTRASCHALLBILD**

(30) Priorität: 09.04.2025 DE 102025113945
(71) Anmelder: B. Braun Melsungen AG, 34212 Melsungen (DE)
(72) Erfinder: Blum, Iris, 34277 Fuldabrück (DE)
(74) Vertreter: Patentanwälte Ruff, Wilhelm, Beier, Dauster & Partner mbB

(57) **Zusammenfassung**

Die Erfindung betrifft ein Verfahren zum Ermitteln einer Position einer Kanüle, insbesondere einer Kanülenspitze und/oder eines Kanülenrohrs, in einem Ultraschallbild, aufweisend die Schritte: Erstellen eines Ultraschallbilds mittels einem bildgebenden Ultraschallsystem; Ermitteln der Position der Kanülenspitze und/oder des Kanülenrohrs in dem Ultraschallbild durch die Kanüle verursachte Gewebebewegungen und/oder Ultraschall-Reflexionen der Kanüle mittels eines trainierten Kl-Systems; und Anzeigen der ermittelten Position der Kanülenspitze und/oder des Kanülenrohrs mittels einer Anzeigeeinrichtung. Die Erfindung betrifft zudem ein Trainingssystem, einen Trainingsdatensatz, ein computerlesbares Speichermedium und ein medizinisches System zum Ermitteln einer Position einer Kanüle.

## Beschreibung

Die Erfindung betrifft ein Verfahren und ein medizinisches System zum Ermitteln einer Position einer Kanülenspitze in einem Ultraschallbild. Die Erfindung bezieht sich auch auf ein Trainingssystem, auf ein computerlesbares Speichermedium und auf einen Trainingsdatensatz für ein Training eines KI-Systems zum Ermitteln einer Position einer Kanülenspitze innerhalb eines Ultraschallbilds.

Bei Ultraschalluntersuchungen werden Ultraschallwellen mittels eines sogenannten Ultraschallkopfs durch ein zu untersuchendes Gewebe gesendet. Die Ultraschallwellen werden an den Grenzen zwischen beispielsweise Organen und Gewebestrukturen in unterschiedlichem Maße reflektiert. Der Ultraschallkopf empfängt die reflektierten Schallwellen, wobei daraus ein Bild errechnet wird, das auf einer Anzeigeeinrichtung, beispielsweise einem Monitor, als Ultraschallbild angezeigt wird.

Ultraschallsysteme kommen beispielsweise bei der Durchführung einer peripheren Nervenblockade zum Einsatz. Bei einer peripheren Nervenblockade muss der behandelnde Anästhesist den zu betäubenden Nerven im Körper des Patienten identifizieren und danach eine Kanüle mit ihrer Kanülenspitze so dicht wie möglich an den Nerven heranbringen. Danach erfolgt die Injektion des Lokalanästhetikums, um die Schmerzweiterleitung zu unterbrechen. Um Gewebestrukturen, insbesondre Nerven, im Körpergewebe des Patienten und die Kanüle zu identifizieren, werden bei der peripheren Nervenblockade und bei vielen weiteren medizinischen Anwendungen bildgebende Ultraschallverfahren unter Verwendung eines Ultraschallkopfs eingesetzt. Dies setzt eine ausreichende Sichtbarkeit der Kanüle im Ultraschallbild voraus. Mit anderen Worten muss die Kanüle eine ausreichende Ultraschallsichtbarkeit aufweisen. Sobald die Zielgewebestruktur, beispielsweise der besagte Nerv, im Ultraschallbild identifiziert ist, punktiert der Anästhesist den Patienten und schiebt die Kanüle so weit vor, bis die Kanülenspitze eine gewünschte Position in Bezug auf die Zielgewebestrukturaufweist, in diesem Beispiel nahe bei dem entsprechenden Nerven positioniert ist, ohne diesen zu berühren. Die Ermittlung einer Position der Kanüle, insbesondere eines Kanülenrohrs und der Kanülenspitze, mittels eines bildgebenden Ultraschallverfahrens stellt sich jedoch als sehr herausfordernd dar, da das Kanülenrohr unter Umständen nur unzureichend dargestellt wird, beispielsweise wenn der Ultraschallkopf nicht parallel zum Kanülenrohr liegt.

Aufgabe der Erfindung ist es, ein Verfahren und ein medizinisches System der eingangs genannten Art bereitzustellen, die Vorteile gegenüber dem Stand der Technik bieten. Im Speziellen soll eine verbesserte visuelle Sichtbarkeit der Kanüle, insbesondere des Kanülenrohrs und der Kanülenspitze, in einem Ultraschallbild und letztlich eine verbesserte Positionskontrolle der Kanüle ermöglicht werden.

Diese Aufgabe wird durch das Bereitstellen eines Verfahrens mit den Merkmalen des Anspruchs 1, eines Trainingssystems mit den Merkmalen des Anspruchs 6, eines medizinischen Systems mit den Merkmalen des Anspruchs 7, eines computerlesbares Speichermediums mit den Merkmalen des Anspruchs 10 und eines Trainingsdatensatzes mit den Merkmalen des Anspruchs 11 gelöst. Vorteilhafte Weiterbildungen sind in den Unteransprüchen angegeben. Der Wortlaut der Ansprüche wird durch Bezugnahme zum Gegenstand der Beschreibung gemacht.

Das erfindungsgemäße Verfahren ist zum Ermitteln einer Position einer Kanülenspitze in einem Ultraschallbild vorgesehen und weist folgende Schritte auf: Erstellen eines Ultraschallbilds mittels eines bildgebenden Ultraschallsystems; Ermitteln der Position der Kanüle, insbesondere der Kanülenspitze und/oder des Kanülenrohrs, in dem Ultraschallbild, vorzugsweise in Bezug auf einen Ultraschallkopf des bildgebenden Ultraschallsystems, in Abhängigkeit von durch die Kanüle verursachten Gewebebewegungen und/oder Ultraschall-Reflexionen der Kanüle mittels eines trainierten KI-Systems (Künstliche Intelligenz-System); und Anzeigen der ermittelten Position der Kanüle, insbesondere der Kanülenspitze und/oder des Kanülenrohrs, mittels einer Anzeigeeinrichtung.

Das Erstellen des Ultraschallbilds erfolgt hierbei mittels eines der Fachperson in Funktion und Aufbau grundsätzlich bekannten, bildgebenden Ultraschallsystems. In einer möglichen Ausführung wird zur Erstellung des Ultraschalbilds mit einem Ultraschallkopf ein Ultraschallsignal in das Körpergewebe eines Patienten eingekoppelt, wobei der Ultraschallkopf beispielsweise auf dem Patientenkörper aufgelegt oder in diesen eingeführt wird. Der Ultraschallkopf empfängt das (reflektierte) Ultraschallsignal und leitet dieses an eine Auswerteeinrichtung weiter, welche das Ultraschalbild erstellt. Dieses Ultraschalbild kann an einer optionalen Anzeigeeinrichtung des Ultraschallsystems angezeigt werden. Das Ultraschallbild wird in dieser Offenbarung auch als Basis-Ultraschallbild bezeichnet.

Bei einer medizinischen Anwendung, wie beispielsweise einer Punktion mit der Kanüle, verursacht die Kanüle auf dem Weg zu ihrer Zielstruktur im Inneren eines Körpers Gewebebewegungen, welche im Ultraschallbild darstellbar bzw. sichtbar sind. Zudem werden in Abhängigkeit der Position und einer Ausrichtung der Kanüle in Bezug auf den Ultraschallkopf die Ultraschallwellen von der Kanüle unterschiedlich reflektiert. Hierdurch wird im Ultraschallbild die Kanüle mit unterschiedlicher Deutlichkeit sichtbar. Das Anzeigen der ermittelten Position der Kanüle kann dabei innerhalb des erstellten Ultraschallbilds beispielsweise durch eine Überlagerung oder in einer dazu separaten Anzeige erfolgen. Das erfindungsgemäße Verfahren hat den Vorteil, dass der Anwender die Kanüle, insbesondere die Kanülenspitze, zielgenau und zuverlässig an der Zielgewebestruktur positionieren kann. Dieses Verfahren ist dabei mit jeder handelsüblichen Kanüle anwendbar. Das Verfahren ist beispielsweise bei der oben genannten peripheren Nervenblockade hilfreich, da es den Anwender, in diesem Fall den Anästhesisten, dabei unterstützt, die Kanüle möglichst nah an dem Nerven zu positionieren. Das erfindungsgemäße Verfahren erlaubt wenigstens ein näherungsweises Ermitteln der Position der Kanüle. Bei einer Ausgestaltung wird ein Axialabstand zwischen der Kanüle, insbesondere der Kanülenspitze, und einer Bildebene ermittelt. Bei einer weiteren Ausgestaltung wird alternativ oder zusätzlich ein Neigungswinkel der Kanüle in einer Vertikalebene ermittelt. Bei einer weiteren Ausgestaltung wird alternativ oder zusätzlich ein Tiefenabstand zwischen dem Ultraschallkopf und der Kanüle ermittelt. Bei einer weiteren Ausgestaltung wird alternativ oder zusätzlich ein Drehwinkel der Kanüle in einer Horizontalebene ermittelt. Anhand einzelner, mehrerer oder aller der vorgenannten Größen kann die Position der Kanüle in Bezug auf den Ultraschallkopf wenigstens näherungsweise ermittelt werden.

In Ausgestaltung der Erfindung erfolgt das Ermitteln der Position der Kanüle, insbesondere der Kanülenspitze und/oder des Kanülenrohrs, zusätzlich in Abhängigkeit einer Markierung in Form eines Markierungsmusters an bzw. auf der Kanüle, insbesondere an dem Kanülenrohr und/oder an der Kanülenspitze. Auch diese Ermittlung erfolgt mittels des trainierten KI-Systems. Dadurch wird die Sichtbarkeit der Kanüle im Ultraschallbild und dadurch auch die Positionsermittlung der Kanüle weiter verbessert. Das Markierungsmuster kann beispielsweise in Form von echogenen Strukturen auf dem Kanülenrohr angebracht sein. Die echogenen Strukturen sind in einem Ultraschallbild deutlich sichtbar. Das Markierungsmuster weist mindestens einen Markierungsabschnitt auf. Die Kanüle weist vorteilhaft mehrere solcher über das Kanülenrohr axial beabstandete Markierungsabschnitte, insbesondere in unterschiedlicher Länge, auf. Diese Markierungsabschnitte weisen vorteilhaft einen definierten axialen Abstand zur Kanülenspitze auf, so dass sich deren Position in Abhängigkeit des axialen Abstands noch genauer ermitteln bzw. berechnen lässt. Das Markierungsmuster kann auch als Markierungssequenz bezeichnet werden. Das Markierungsmuster ermöglicht eine eindeutige Lokalisierung der Kanülenspitze, selbst wenn die Kanüle im Ultraschallbild nicht vollständig darstellbar ist. Zusätzlich kann die Kanüle ein Ortungselement beispielsweise in Form eines Sensors aufweisen, in dessen Abhängigkeit zusätzlich das Ermitteln der Position der Kanüle erfolgt.

In weiterer Ausgestaltung der Erfindung umfasst das Markierungsmuster mehrere axial voneinander beabstandete Markierungsabschnitte. Das Markierungsmuster kann beispielsweise in distaler Richtung einen längeren Markierungsabschnitt gefolgt von zwei bezogen auf den längeren Markierungsabschnitt kürzere Markierungsabschnitte umfassen. Die Länge der Markierungsabschnitte ist hierbei definiert als die Ausdehnung in axialer Richtung entlang der Kanüle. Das Markierungsmuster kann auch nur zwei oder mehr als zwei Markierungsabschnitte aufweisen, vorteilhaft in unterschiedlichen axialen Längen.

In weiterer Ausgestaltung der Erfindung umfasst das Ermitteln der Position ein Erstellen eines modifizierten Ultraschallbilds durch das trainierte KI-System. Dabei legt das trainierte KI-System in dem modifizierten Ultraschallbild schematisch eine Linie über das Kanülenrohr und/oder einen Punkt auf die Kanülenspitze. Das modifizierte Ultraschallbild kann somit ein schematisch vereinfachtes Abbild der Kanüle aufweisen. Dies stellt eine funktional vorteilhafte Realisierung der Erfindung dar, indem dadurch die visuelle Sichtbarkeit der Kanüle, insbesondere des Kanülenrohrs und der Kanülenspitze, und somit auch die Positionskontrolle der Kanüle weiter verbessert wird. Das modifizierte Ultraschallbild wird vorzugsweise mittels der Anzeigeeinrichtung angezeigt. Das modifizierten Ultraschallbild kann beispielsweise auf einem separaten Monitor oder in einem zweiten Anzeigefenster angezeigt werden. Alternativ kann das modifizierte Ultraschallbild auch über das Ultraschallbild gelegt werden, wobei das Kanülenrohr in Form der Linie und/oder die Kanülenspitze in Form des Punkts klar und eindeutig abgegrenzt wird.

In weiterer Ausgestaltung der Erfindung umfasst das Ermitteln der Position weiter ein Angeben von Eigenschaften der eingesetzten Kanüle. Die Eigenschaften der Kanüle können beispielsweise die Art und Anordnung der Markierungen auf der Kanüle und/oder die Abmessungen der Kanüle sein. Das Angeben der Eigenschaften der Kanüle findet vorzugsweise zu Beginn dieses Schrittes statt. Dadurch wird der durch das trainierte KI-System abzugleichende Datensatz verkleinert, wodurch sich im nächsten Schritt das Ermitteln der Position der Kanüle, im Speziellen der Kanülenspitze, innerhalb des erstellten Ultraschallbilds durch das trainierte Kl-System verschnellert und verbessert. Das Ermitteln der Position umfasst zudem ein Bereitstellen von die Kanüle betreffenden technischen Zeichnungen und/oder daraus abgeleiteten Referenzdaten, wobei die angegebenen und/oder bereitgestellten Eigenschaften und die technischen Zeichnungen und/oder Referenzdaten zum Parametrieren und/oder Trainieren eines lernbasierten Auswertealgorithmus, insbesondere eines Kl-Modells, verwendet werden, der/das zur Durchführung des Ermittelns herangezogen wird. Vorteilhaft wird zur Ermittlung der Position die aktuelle Ausrichtung und Länge der Kanüle herangezogen.

Das erfindungsgemäße Trainingssystem zum Ermitteln einer Position einer Kanüle, insbesondere einer Kanülenspitze und/oder eines Kanülenrohrs, in einem Ultraschallbild umfasst einen Ultraschallkopf eines bildgebenden Ultraschallsystems, wobei der Ultraschallkopf einerseits durch eine Ortungskanüle verursachte Gewebebewegungsdaten und/oder Ultraschall-Reflexionsdaten der Ortungskanüle erfasst und einer Steuereinheit bereitstellt und andererseits dazu eingerichtet ist, eine Position der Ortungskanüle zu erfassen und der Steuereinheit bereitzustellen. Die Ortungskanüle weist vorzugsweise eine im Vergleich zu herkömmlichen Kanülen verbesserte Sichtbarkeit in einem Ultraschallbild auf. Das Trainingssystem umfasst weiter die Steuereinheit, welche die erfassten Gewebebewegungsdaten und/oder Ultraschall-Reflexionsdaten der Ortungskanüle mit der erfassten Position der Ortungskanüle verknüpft, um einen Trainingsdatensatz zu erhalten. Die Ortungskanüle ist dazu eingerichtet, mittels des bildgebenden Ultraschallsystems geortet zu werden. Zudem umfasst das Trainingssystem die Kanüle betreffende technische Zeichnungen und/oder daraus abgeleitete Referenzdaten, wobei die angegebenen und/oder bereitgestellten Eigenschaften und die technischen Zeichnungen und/oder Referenzdaten zum Parametrieren und/oder Trainieren eines lernbasierten Auswertealgorithmus, insbesondere eines Kl-Modells, verwendet werden, der/das zur Durchführung des Ermittelns herangezogen wird.

Das erfindungsgemäße medizinische System zum Ermitteln einer Position einer Kanüle, insbesondere einer Kanülenspitze und/oder eines Kanülenrohrs, in einem Ultraschallbild umfasst ein bildgebendes Ultraschallsystem mit einem Ultraschallkopf und einer Auswerteeinrichtung. Das Ultraschallsystem ist hinsichtlich Funktion und Aufbau einer Fachperson grundsätzlich bekannt, so dass auf diesbezügliche Merkmale des Ultraschallsystems nicht im Detail weiter eingegangen werden braucht. Der Ultraschallkopf des Ultraschallsystems ist zum Einkoppeln eines Ultraschallsignals in Gewebe, insbesondere Körpergewebe und/oder den Körper eines Patienten, eingerichtet. Dazu wird der Ultraschallkopf beispielsweise auf den Körper des Patienten aufgelegt oder in diesen eingeführt. Die Auswerteeinrichtung ist dazu eingerichtet, ein Ultraschallbild basierend auf einem empfangenen Ultraschallsignal zu erstellen. Das erfindungsgemäße medizinische System umfasst weiter ein trainiertes KI-System, das dazu ausgebildet ist, basierend auf dem empfangenen Ultraschallsignal und einem statistischen Modell, die Kanüle, insbesondere die Kanülenspitze und/oder das Kanülenrohr, innerhalb des erstellten Ultraschallbilds zu identifizieren, wobei das trainierte KI-System bzw. das statistische Modell mittels Trainingsdaten trainiert worden ist. Das trainierte KI-System ist vorzugsweise auf einem computerlesbaren Speichermedium gespeichert und kann dann zusammen mit dem bildgebenden Ultraschallsystem für eine Anwendung in einem medizinischen Bereich eingesetzt werden, insbesondere bei einer Punktion. Die Kanüle weist, insbesondere an dem Kanülenrohr und/oder an der Kanülenspitze, eine Markierung in Form eines Markierungsmusters auf, das eine eindeutige Lokalisierung der Kanülenspitze ermöglicht, selbst wenn die Kanüle im Ultraschallbild nicht vollständig darstellbar ist.

Das KI-System wird vorzugsweise als ein künstliches neuronales Netz, insbesondere ein Recurrent Neural Network (RNN), besonders bevorzugt ein Long Short Term Memory Network (LSTM-Netz) und/oder ein Convolutional Neural Network (CNN), verwendet. Hierdurch kann insbesondere eine Tiefe des neuronalen Netzwerks angepasst werden und ein Training so lange durchgeführt werden, bis eine hinreichend genaue Gewichtung erreicht ist.

**In** Ausgestaltung der Erfindung ist das trainierte KI-System weiter dazu ausgebildet, ein dem Ultraschallbild entsprechendes, modifiziertes Ultraschallbild zu erstellen. In dem modifizierten Ultraschallbild legt das trainierte KI-System eine Linie über das Kanülenrohr und/oder einen Punkt auf die Kanülenspitze. Dabei erfolgt die Einblendung der Linie und/oder des Punktes auch dann, wenn das Kanülenrohr und/oder die Kanülenspitze im ursprünglichen Ultraschallbild nur teilweise sichtbar sind.

In weiterer Ausgestaltung der Erfindung weist das medizinische System weiter eine Anzeigeeinrichtung auf, die zur Anzeige des Basis-Ultraschallbilds und/oder des modifizierten Ultraschallbilds eingerichtet ist. Die Anzeigeeinrichtung ist beispielsweise ein Monitor, auf welchem sowohl das Basis-Ultraschallbild als auch das modifizierte Ultraschallbild darstellbar ist. Das modifizierte Ultraschallbild kann dabei zusätzlich oder alternativ zu dem Basis-Ultraschallbild angezeigt werden oder diesem überlagert sein. Das Basis-Ultraschallbild und das modifizierte Ultraschallbild werden im Wesentlichen unverzögert auf der Anzeigeeinrichtung angezeigt, so dass es beispielsweise dem Anwender bei einer peripheren Nervenblockade ermöglicht, die Kanüle möglichst nah an dem Nerven zu platzieren.

Das erfindungsgemäße computerlesbare Speichermedium umfasst Befehle, die bei einer Ausführung durch einen Computer diesen veranlassen, die Verfahrensschritte des erfindungsgemäßen Verfahrens auszuführen.

Der erfindungsgemäße Trainingsdatensatz für eine Ermittlung einer Position einer Kanüle, insbesondere einer Kanülenspitze und/oder eines Kanülenrohrs, in einem Ultraschallbild umfasst einerseits die erfassten, durch die Ortungskanüle verursachten Gewebebewegungsdaten und/oder die erfassten Ultraschall-Reflexionsdaten der Ortungskanüle, andererseits zu jeder dieser Daten eine zugehörige erfasste Position der Ortungskanüle. Die Basis-Ultraschallbilder sind mit den entsprechenden Gewebebewegungsdaten und/oder Ultraschall-Reflexionsdaten der Ortungskanüle zugeordnet bzw. werden von einer Steuereinheit verknüpft, so dass ein verknüpfter (Trainings-)Datensatz entsteht. Der verknüpfte Trainingsdatensatz kann für das Training bzw. das Anlernen eines KI-Systems herangezogen werden. Insbesondere kann ein computerlesbares Speichermedium den Trainingsdatensatz gemäß der vorliegenden Ausführungsform aufweisen.

Ein Grundgedanke der vorliegenden Offenbarung ist demnach, durch das Trainingssystem eine Verknüpfung zwischen den erstellten Basis-Ultraschallbildern des bildgebenden Ultraschallsystems und den erfassten Gewebebewegungsdaten und/oder Ultraschall-Reflexionsdaten der Ortungskanüle herzustellen. Das trainierte KI-System ist in der Lage als eine Art Wandler zwischen einer Eingabe und einer Ausgabe, die Basis-Ultraschallbilder als Eingabe in modifizierte Ultraschallbilder als Ausgabe umzuwandeln und damit die Position der Kanüle, im Speziellen der Kanülenspitze und/oder des Kanülenrohrs, zu ermitteln und anzuzeigen.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in Blockdarstellung eine Ausführungsform eines erfindungsgemäßen medizinischen Systems zum Ermitteln einer Position einer Kanüle in einem Ultraschallbild,
- Fig. 2: in schematischer Blockdarstellung eine Ausführungsform eines erfindungsgemäßen Verfahrens zum Ermitteln einer Position einer Kanüle in einem Ultraschallbild,
- Fig. 3A: ein exemplarisches Ultraschallbild, welches Gewebestrukturen und abschnittsweise eine Kanüle mit einem Kanülenrohr und einer Kanülenspitze zeigt,
- Fig. 3B: ein exemplarisches modifiziertes Ultraschallbild, welches das Kanülenrohr in Form einer Linie und die Kanülenspitze in Form eines Punkts der im Ultraschallbild nach Fig. 3A abschnittsweise gezeigten Kanüle anzeigt,
- Fig. 4: eine Überlagerung des Ultraschallbilds nach Fig. 3A und des modifizierten Ultraschallbilds nach 3B,
- Fig. 5: in schematischer Blockdarstellung eine Ausführungsform eines Trainingssystems zum Trainieren eines KI-Systems, und
- Fig. 6: in schematischer Seitenansicht eine weitere Ausführungsform einer Kanüle aufweisend ein Markierungsmuster.

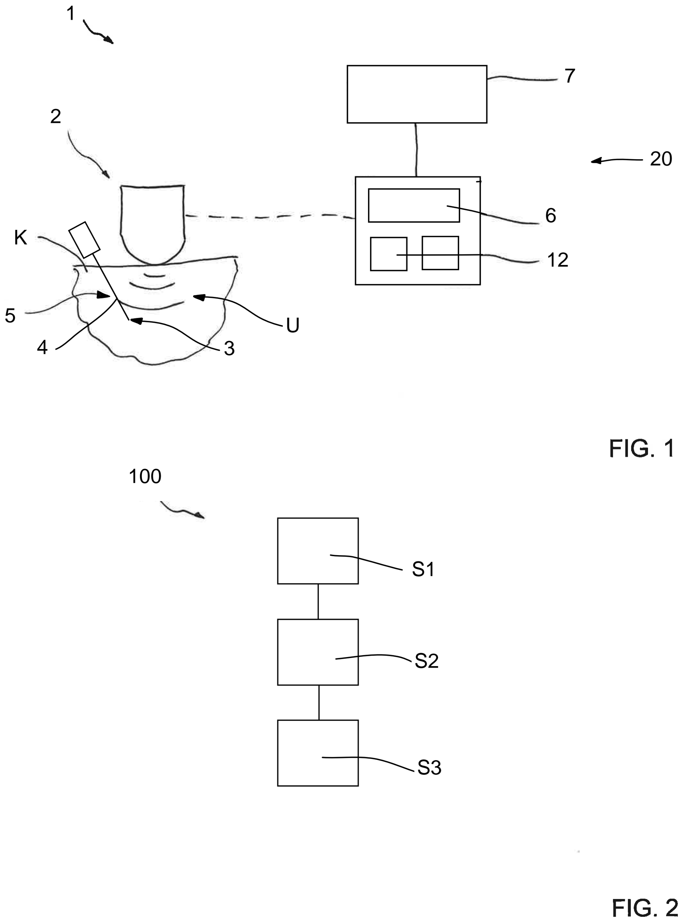

Gemäß Fig. 2 ist ein Verfahren 100 zum Ermitteln einer Position einer Kanüle 5 in einem Ultraschallbild A vorgesehen. Vorliegend ist das Verfahren 100 zum Ermitteln einer Position einer Kanülenspitze 3 und eines Kanülenrohrs 4 der Kanüle 5 im Inneren eines Körpers K eines Patienten eingerichtet. Das Verfahren 100 kann mit einem in Fig. 1 gezeigten medizinischen System 1 ausgeführt werden, beispielsweise bei einer peripheren Nervenblockade.

Das medizinische System 1 weist vorliegend ein bildgebendes Ultraschallsystem 20 mit einem Ultraschallkopf 2, einer Auswerteeinrichtung 12 und einer optionalen Anzeigeeinrichtung 7 auf, wie in Fig. 1 gezeigt ist. Die Auswerteeinrichtung 12 ist dazu eingerichtet, ein (Basis-)Ultraschallbild A basierend auf einem empfangenen Ultraschallsignal U zu erstellen. Die Anzeigeeinrichtung 7 ist zur Anzeige des Ultraschallbilds A eingerichtet.

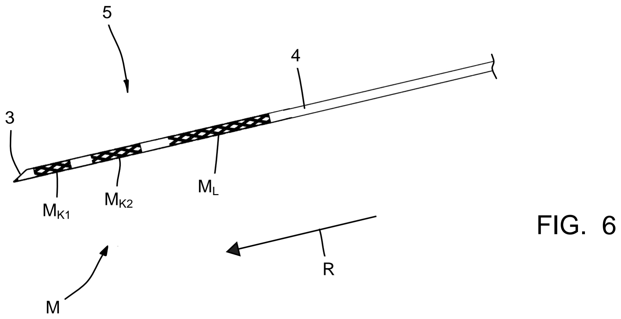

Das medizinische System 1 weist weiter ein trainiertes KI-System 6 auf. Das trainierte KI-System 6 ist dazu ausgebildet, basierend auf dem empfangenen Ultraschallsignal U bzw. basierend auf dem daraus erstellten Ultraschallbild A und einem statistischen Modell, die Kanüle 5 innerhalb des erstellten Ultraschallbilds A zu identifizieren. Dabei ist das KI-System 6 mittels Trainingsdaten trainiert worden. Die Kanüle 5 weist beispielsweise, insbesondere an dem Kanülenrohr 4 und/oder an der Kanülenspitze 3, eine Markierung in Form eines Markierungsmusters M auf, wie in Fig. 6 gezeigt ist. Das Markierungsmuster M ermöglicht eine eindeutige Lokalisierung der Kanülenspitze 3, selbst wenn die Kanüle 5 im Ultraschallbild A nicht vollständig darstellbar ist.

In einer vorteilhaften Ausführung weist das Markierungsmuster M mehrere axial voneinander beabstandete Markierungsabschnitte M_{L}, M_{K1}, M_{K2} auf. Das Markierungsmuster M kann beispielsweise in distaler Richtung R einen ersten Markierungsabschnitt M_{L} gefolgt von zwei weiteren Markierungsabschnitten M_{K1}, M_{K2} aufweisen, wobei der Markierungsabschnitt M_{L} bezogen auf die zwei weiteren Markierungsabschnitte M_{K1}, M_{K2} länger ist, wie in Fig. 6 gezeigt ist.

In nicht gezeigten Ausführungen weist das Markierungsmuster M an der Kanüle 5 nur zwei oder mehr als drei Markierungsabschnitte auf, vorteilhaft in unterschiedlichen axialen Längen.

Der Schritt S1 des Verfahrens 100 sieht ein Erstellen eines Ultraschallbilds A mittels des bildgebenden Ultraschallsystems 20 vor. Das Ultraschallsignal U wird mit dem Ultraschallkopf 2 abgegeben und in den Körper K des Patienten eingekoppelt. In der gezeigten Ausführungsform (Fig. 1) liegt der Ultraschallkopf 2 auf dem Körper K auf. Alternativ kann der Ultraschallkopf 2 auch in eine Öffnung des Körpers K des Patienten eingeführt und somit im Inneren des Körpers K angeordnet sein. Das Ultraschallsignal U wird von unterschiedlichen Gewebestrukturen und von der Kanüle 5 je nach Position und Ausrichtung in Bezug auf den Ultraschallkopf 2 unterschiedlich stark reflektiert. Der Ultraschallkopf 2 empfängt das reflektierte Ultraschallsignal und leitet dieses an die Auswerteeinrichtung 12 weiter, welche aus dem reflektierten Ultraschallsignal das Ultraschallbild A erstellt. Das Ultraschallbild A kann an der Anzeigeeinrichtung 7 angezeigt werden, wie in Fig. 1 gezeigt ist.

In einer vorteilhaften Ausführung werden zu Beginn des Schritts S1 die Eigenschaften der verwendeten Kanüle 5 angegeben. Dies können beispielsweise die Abmessungen der Kanüle 5 und/oder Angaben über Markierungen auf der Kanüle 5 sein. Dadurch wird der durch das trainierte KI-System 6 abzugleichende Datensatz verkleinert.

Der Schritt S2 des Verfahrens 100 sieht ein Ermitteln der Position der Kanüle 5 bzw. der Kanülenspitze 3 und des Kanülenrohrs 4 in dem Ultraschallbild A in Abhängigkeit von durch die Kanüle 5 verursachten Gewebebewegungen und/oder Ultraschall-Reflexionen der Kanüle 5 mittels des trainierten KI-Systems 6 des medizinischen Systems 1 vor. In einer vorteilhaften Ausführung erfolgt dies zusätzlich in Abhängigkeit einer Markierung in Form eines Markierungsmusters M an der Kanüle 5, insbesondere an dem Kanülenrohr 4 und/oder an der Kanülenspitze 3. Das KI-System 6 ist derart trainiert, dass es die Position der in dem Ultraschallbild A vorhandenen Kanüle 5 ermittelt.

In einer vorteilhaften Ausführung werden bei Schritt S2 die Kanüle betreffende technische Zeichnungen und/oder daraus abgeleitete Referenzdaten bereitgestellt, wobei die angegebenen und/oder bereitgestellten Eigenschaften und die technischen Zeichnungen und/oder Referenzdaten zum Parametrieren und/oder Trainieren eines lernbasierten Auswertealgorithmus, insbesondere eines KI-Modells, verwendet werden, der/das zur Durchführung des Ermittelns S2 herangezogen wird.

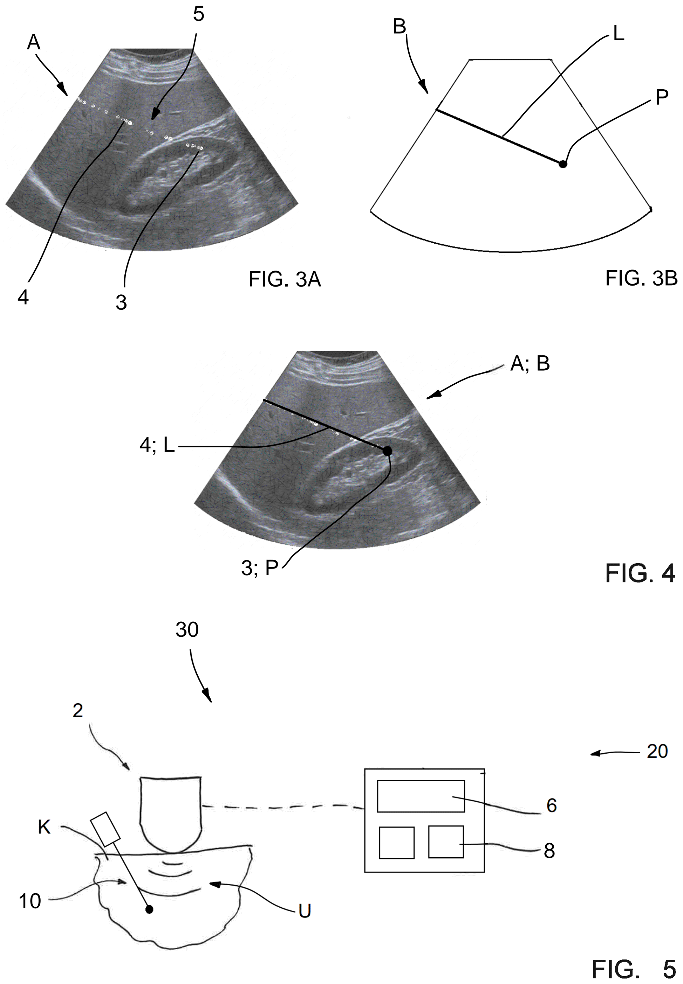

Um das trainierte KI-System 6 zu erhalten, ist ein Trainingssystem 30 gemäß Fig. 5 vorgesehen. Das Trainingssystem 30 umfasst einen Ultraschallkopf, beispielsweise den Ultraschallkopf 2 des bildgebenden Ultraschallsystems 20. Dabei ist der Ultraschallkopf 2 dazu eingerichtet, in Abhängigkeit von durch eine Ortungskanüle 10 verursachten Gewebebewegungsdaten und Ultraschall-Reflexionsdaten der Ortungskanüle 10 zu erfassen und einer Steuereinheit 8 bereitzustellen. Zudem ist der Ultraschallkopf 2 dazu eingerichtet, eine Position der Ortungskanüle 10 zu erfassen und ebenfalls der Steuereinheit 8 bereitzustellen. Das Trainingssystem 30 umfasst weiter die Steuereinheit 8, welche die erfassten Gewebebewegungsdaten und Ultraschall-Reflexionsdaten der Ortungskanüle 10 mit der erfassten Position der Ortungskanüle 10 verknüpft, um einen Trainingsdatensatz zu erhalten. Der Trainingsdatensatz umfasst somit die erfassten Gewebebewegungen und Ultraschall-Reflexionen der Ortungskanüle 10 sowie die jeweils dazugehörige erfasste Position der Ortungskanüle 10. Das Trainingssystem 30 umfasst zudem die Kanüle 5 betreffende technische Zeichnungen und/oder daraus abgeleitete Referenzdaten, wobei die angegebenen und/oder bereitgestellten Eigenschaften und die technischen Zeichnungen und/oder Referenzdaten zum Parametrieren und/oder Trainieren eines lernbasierten Auswertealgorithmus, insbesondere eines Kl-Modells, verwendet werden, der/das zur Durchführung des Ermittelns S2 herangezogen wird. Zudem können zur Erzeugung eines Trainingsdatensatzes mehrere unterschiedliche Ortungskanülen 10 eingesetzt werden, um einen möglichst breiten Trainingsdatensatz zu erhalten, welcher alle auf dem Markt üblichen Kanülen abdeckt.

In einer vorteilhaften Ausführung wird durch das trainierte KI-System 6 ein modifiziertes Ultraschallbild B erstellt. Das trainierte KI-System 6 legt vorliegend in dem modifizierten Ultraschallbild B schematisch eine Linie L über das Kanülenrohr 4 und einen Punkt P auf die Kanülenspitze 3, wie in der Fig. 3B gezeigt ist. Dabei kann die Einblendung der Linie L und/oder des Punktes P auch dann erfolgen, wenn das Kanülenrohr 4 und/oder die Kanülenspitze 3 im ursprünglichen Ultraschallbild A nur teilweise sichtbar sind. Das trainierte KI-System 6 des medizinischen Systems 1 ist somit dazu ausgebildet, ein dem Ultraschallbild A entsprechendes, modifiziertes Ultraschallbild B zu erstellen, welches ein schematisches Abbild der innerhalb des Ultraschallbilds A vorhandenen Kanüle 5 darstellt.

Das Verfahren 100 sieht in dem Schritt S3 ein Anzeigen der ermittelten Position der Kanüle 5, vorliegend die Position der Kanülenspitze 3 und des Kanülenrohrs 4, mittels der Anzeigeeinrichtung 7 vor. Wird die Kanüle 5 unter Verwendung eines bildgebenden Ultraschallverfahrens im Körpergewebe positioniert, kann das ausführende medizinische Personal in dem sich ergebenden Ultraschallbild einfach und zuverlässig zwischen dem Kanülenrohr 4 und der Kanülenspitze 3 differenzieren. Dies erlaubt eine verbesserte Positionierung der Kanüle 5 in dem Körpergewebe. Vorliegend wird das modifizierte Ultraschallbild B (Fig. 3B) dem Basis-Ultraschallbild A (Fig. 3A) überlagert, so dass beide Ultraschallbilder A, B in einem gemeinsamen Ultraschallbild dargestellt werden, wie in Fig. 4 gezeigt ist. In einer alternativen Ausführung werden das Basis-Ultraschallbild A und das modifizierte Ultraschallbild B nebeneinander mittels der Anzeigeeinrichtung 6 angezeigt.

## Patentansprüche

1. Verfahren (100) zum Ermitteln einer Position einer Kanüle (5), insbesondere einer Kanülenspitze (3) und/oder eines Kanülenrohrs (4), in einem Ultraschallbild (A), aufweisend die Schritte:
- Erstellen (S1) eines Ultraschallbilds (A) mittels einem bildgebenden Ultraschallsystem (20);
- Ermitteln (S2) der Position der Kanüle (5), insbesondere der Kanülenspitze (3) und/oder des Kanülenrohrs (4), in dem Ultraschallbild (A) in Abhängigkeit von durch die Kanüle (5) verursachte Gewebebewegungen und/oder Ultraschall-Reflexionen der Kanüle (5) mittels eines trainierten KI-Systems (6); und
- Anzeigen (S3) der ermittelten Position der Kanüle (5), insbesondere der Kanülenspitze (3) und/oder des Kanülenrohrs (4), mittels einer Anzeigeeinrichtung (7).

2. Verfahren (100) nach Anspruch 1,
- wobei das Ermitteln (S2) der Position der Kanüle (5), insbesondere der Kanülenspitze (3) und/oder des Kanülenrohrs (4), zusätzlich in Abhängigkeit einer Markierung in Form eines Markierungsmusters (M) an der Kanüle (5), insbesondere an dem Kanülenrohr (4) und/oder an der Kanülenspitze (3), erfolgt.

3. Verfahren (100) nach Anspruch 2,
- wobei das Markierungsmuster (M) mehrere axial voneinander beabstandete Markierungsabschnitte (M_{L}, M_{K1}, M_{K2}) umfasst, insbesondere in distaler Richtung (R) einen ersten Markierungsabschnitt (M_{L}) gefolgt von wenigstens einem weiteren Markierungsabschnitt (M_{K1}, M_{K2}), wobei der erste Markierungsabschnitt (M_{L}) länger ist als der wenigstens eine weitere Markierungsabschnitt (M_{K1}, M_{K2}).

4. Verfahren (100) nach einem der Ansprüche 1 bis 3, wobei das Ermitteln (S2) der Position weiter umfasst:
- Erstellen eines modifizierten Ultraschallbilds (B) durch das trainierte KI-System (6), wobei das trainierte KI-System (6) in dem modifizierten Ultraschallbild (B) schematisch eine Linie (L) über das Kanülenrohr (4) und/oder einen Punkt (P) auf die Kanülenspitze (3) legt.

5. Verfahren (100) nach einem der Ansprüche 1 bis 4, wobei das Ermitteln (S2) der Position weiter umfasst:
- Angeben von Eigenschaften, insbesondere Abmessungen und/oder Markierungen, der eingesetzten Kanüle (5), und
- Bereitstellen von die Kanüle (5) betreffenden technischen Zeichnungen und/oder daraus abgeleiteten Referenzdaten, wobei die angegebenen und/oder bereitgestellten Eigenschaften und die technischen Zeichnungen und/oder Referenzdaten zum Parametrieren und/oder Trainieren eines lernbasierten Auswertealgorithmus, insbesondere eines KI-Modells, verwendet werden, der/das zur Durchführung des Ermittelns (S2) herangezogen wird.

6. Trainingssystem (30) zum Trainieren eines KI-Systems zum Ermitteln einer Position einer Kanüle (5), insbesondere einer Kanülenspitze (3) und/oder eines Kanülenrohrs (4), in einem Ultraschallbild (A), aufweisend:
- einen Ultraschallkopf (2) eines bildgebenden Ultraschallsystems (20), wobei der Ultraschallkopf (2) einerseits durch eine Ortungskanüle (10) verursachte Gewebebewegungsdaten und/oder Ultraschall-Reflexionsdaten der Ortungskanüle (10) erfasst und einer Steuereinheit (8) bereitstellt und andererseits dazu eingerichtet ist, eine Position der Ortungskanüle (10) zu erfassen und der Steuereinheit (8) bereitzustellen,
- die Ortungskanüle (10),
- die Steuereinheit (8), welche die erfassten Gewebebewegungsdaten und/oder Ultraschall-Reflexionsdaten der Ortungskanüle (10) mit der erfassten Position der Ortungskanüle (10) verknüpft, um einen Trainingsdatensatz zu erhalten, und
- die Kanüle (5) betreffende technische Zeichnungen und/oder daraus abgeleitete Referenzdaten, wobei die angegebenen und/oder bereitgestellten Eigenschaften und die technischen Zeichnungen und/oder Referenzdaten zum Parametrieren und/oder Trainieren eines lernbasierten Auswertealgorithmus, insbesondere eines Kl-Modells, verwendet werden, der/das zur Durchführung des Ermittelns (S2) herangezogen wird.

7. Medizinisches System (1) zum Ermitteln einer Position einer Kanüle (5), insbesondere einer Kanülenspitze (3) und/oder eines Kanülenrohrs (4), in einem Ultraschallbild (A), aufweisend:
- ein bildgebendes Ultraschallsystem (20) mit einem Ultraschallkopf (2) und einer Auswerteeinrichtung (12), die dazu eingerichtet ist, ein Ultraschallbild (A) basierend auf einem empfangenen Ultraschallsignal (U) zu erstellen; und
- ein trainiertes KI-System (6), das dazu ausgebildet ist, basierend auf dem empfangenen Ultraschallsignal (U) und einem statistischen Modell, die Kanüle (5), insbesondere die Kanülenspitze (3) und/oder das Kanülenrohr (4), innerhalb des erstellten Ultraschallbilds (A) zu identifizieren, wobei das statistische Modell mittels Trainingsdaten trainiert worden ist,
- wobei die Kanüle (5), insbesondere an dem Kanülenrohr (4) und/oder an der Kanülenspitze (3), eine Markierung in Form eines Markierungsmusters (M) zur eindeutigen Lokalisierung der Kanülenspitze (3) aufweist.

8. Medizinisches System (1) nach Anspruch 7,
- wobei das trainierte KI-System (6) weiter dazu ausgebildet ist, ein dem Ultraschallbild (A) entsprechendes, modifiziertes Ultraschallbild (B) zu erstellen, in welchem das trainierte KI-System (6) eine Linie (L) über das Kanülenrohr (4) und/oder einen Punkt (P) auf die Kanülenspitze (3) legt,
- wobei die Einblendung der Linie (L) und/oder des Punktes (P) auch dann erfolgt, wenn das Kanülenrohr (4) und/oder die Kanülenspitze (3) im ursprünglichen Ultraschallbild (A) nur teilweise sichtbar sind.

9. Medizinisches System (1) nach Anspruch 7 oder 8, wobei das medizinische System weiter eine Anzeigeeinrichtung (7) aufweist, die zum Anzeigen des Ultraschallbilds (A) und/oder des modifizierten Ultraschallbilds (B) eingerichtet ist.

10. Computerlesbares Speichermedium, das Befehle umfasst, die bei einer Ausführung durch einen Computer diesen veranlassen, die Verfahrensschritte des Verfahrens gemäß einem der Ansprüche 1 bis 5 auszuführen.

11. Trainingsdatensatz für ein Training eines KI-Systems zum Ermitteln einer Position einer Kanüle (5), insbesondere einer Kanülenspitze (3) und/oder eines Kanülenrohrs (4), in einem Ultraschallbild (A), insbesondere ein Trainingsdatensatz eines Trainingssystems (10) nach Anspruch 6, aufweisend:
- erfasste, durch die Kanüle (5) verursachte Gewebebewegungsdaten und/oder Ultraschall-Reflexionsdaten der Kanüle (5), und
- einer zugehörigen erfassten Position der Kanüle (5).
